(19) Europäisches Patentamt

European Patent Office

Office européen des brevets

(11) Veröffentlichungsnummer: **0 214 564**
**A2**

# (12) EUROPÄISCHE PATENTANMELDUNG

(21) Anmeldenummer: 86111946.9

(22) Anmeldetag: 29.08.86

(51) Int. Cl.⁴: **C12N 1/38** , C12P 21/02

(30) Priorität: 10.09.85 DE 3532134

(43) Veröffentlichungstag der Anmeldung:
18.03.87 Patentblatt 87/12

(84) Benannte Vertragsstaaten:
AT BE CH DE FR GB IT LI NL

(71) Anmelder: BASF Aktiengesellschaft
Carl-Bosch-Strasse 38
D-6700 Ludwigshafen(DE)

(72) Erfinder: Biohm, Dietmar, Dr.
Dackenheimer Strasse 25
D-6700 Ludwigshafen(DE)
Erfinder: Martin, Christoph, Dr.
Kolpingstrasse 6
D-6800 Mannheim 1(DE)
Erfinder: Hillen, Heinz, Dr.
Fasanenstrasse 10
D-6700 Ludwigshafen(DE)
Erfinder: Marcinowski, Stefan, Dr.
Weimarer Strasse 82
D-6700 Ludwigshafen(DE)

(54) Verfahren zur Herstellung von Proteinen bestimmter N-terminaler Struktur mit Hilfe von Prokaryonten.

(57) Verfahren zur Herstellung von Proteinen durch Züchtung gegebenenfalls gentechnologisch veränderter prokaryotischer Mikroorganismen und Gewinnung des Zielproteins aus der Biomasse, wobei das gewünschte Protein am N-terminalen Ende hinsichtlich der An-oder Abwesenheit der Aminosäure Methionin dadurch definiert wird, daß man die Züchtung in Gegenwart einer definierten Konzentration an Mangan-oder anderen Schwermetallionen vornimmt.

EP 0 214 564 A2

## Verfahren zur Herstellung von Proteinen bestimmter N-terminaler Struktur mit Hilfe von Prokaryonten

Die Erfindung betrifft ein Verfahren, mit dem es möglich ist, rekombinante und native Proteine aus Prokaryonten zu gewinnen, die je nach gewählten Bedingungen am N-terminalen Ende methioniert oder frei von Methionin (Met) sind.

In prokaryotischen Organismen wie Bakterien und Blaualgen beginnt die Synthese eines jeden Proteins an dessen N-terminalem Ende, und zwar immer mit der Aminosäure Met. Es wird angenommen, daß dieser generelle Mechanismus der prokaryotischen Proteinsynthese dadurch zustande kommt, daß zur Erkennung des sogenannten Translationsstarts für das Anheften der Ribosomen an die m-RNA stets das Codon AUG erforderlich ist, welches Met kodiert. Das gilt auch dann, wenn das reife, aktive oder auf irgendeine Weise prozessierte Protein später aus irgendwelchen Gründen kein Met am N-Terminus mehr enthält.

Die Details der Synthese der Proteine sind gut bekannt (Adams, J.M. and Capecchi, M.R. Proc. Nat. Acad. Sci. U.S. $\underline{55}$ , 147-150). Der Mechanismus der Met-Abspaltung nach beendeter Synthese des Proteinmoleküls ist bei Prokaryonten jedoch weitgehend unbekannt (vgl. Livingston, D.M. and Leder, P. Biochemistry, $\underline{8}$, 435-442 (1969), Brown, J.L. and Krall, J.F. Biochem. and Biophys. Res., Comm. $\underline{42}$, 390-397 (1971)). Eine Methode zur Steuerung dieser Aktivität, d.h. zur Gewinnung von Proteinen mit definierter N-terminaler Struktur ist bisher nicht bekannt geworden.

Eine solche Methode ist aber vor allem dann wünschenswert, wenn prokaryotische Organismen, wie z.B. E. coli, als Wirtsorganismus für gentechnologische Konstruktionen genutzt werden, um mit ihrer Hilfe große Mengen bestimmter Proteine herzustellen.

Es gibt zahlreiche Beispiele dafür, daß auf diesem Wege hergestellte Proteine das N-terminale Met enthalten, obwohl das native, d.h. aus der ursprünglichen biologischen Quelle isolierte Protein am N-Terminus Met-frei ist. Für die Herstellung z.B. von pharmazeutisch wirksamen Proteinen entstehen dadurch Probleme, weil nicht bekannt ist, ob solche "unnatürlichen", am N-Terminus methionierten Proteine zu immunologischen Komplikationen führen. Außerdem entstehen bei Proteinen, die aus einem Aggregat aus mehreren gleichen Proteinketten ("Monomeren") bestehen, wie z.B. der λ-Repressur, zusätzliche Unsicherheiten, wenn die Met-Abspaltung durch die zelluläre Aktivität nur teilweise erfolgt. In diesem Fall können sich Gemische unterschiedlicher Proteinaggregate bilden, deren chemische und biologische Eigenschaften - schwer exakt zu definieren sind.

Eine bekannte Lösung dieses Problems besteht darin, das isolierte Protein mit einem spezifischen, das endständige Met entfernenden Enzym zu behandeln (Blumberg et al. World Biotech. Rep. $\underline{3}$, 120-124 (1985)). Ein solches Verfahren hat eine Reihe von Nachteilen: Es ist nicht nur sehr teuer, sondern bringt vor allem auch eine unerwünschte Verunreinigung und möglicherweise eine Schädigung des in der Regel hochgereinigten Proteins mit sich.

Ziel und Aufgabe der Erfindung bestand deshalb darin, die Aktivität, die innerhalb des prokaryotischen Organismus die Abspaltung des N-terminalen Met bewerkstelligt, so zu steuern, daß das gebildete Protein N-terminal möglichst viel oder (für die meisten Zwecke) möglichst wenig Met enthält.

Gegenstand der Erfindung ist ein Verfahren zur Herstellung von Proteinen durch Züchtung gegebenenfalls gentechnologisch veränderter prokaryotischer Mikroorganismen und Gewinnung des Proteins aus der Biomasse, wobei das gewünschte Protein N-terminal möglichst viel oder wenig Met enthält, welches darin besteht, daß man die Züchtung der Mikroorganismen je nach dem gewünschten Methioningehalt bei einer im Fermentationsmedium gezielt im Bereich von $10^{-2}$ bis unter $10^{-6}$ mol/l eingestellten Konzentration an Schwermetallionen vornimmt. Bei Konzentrationen außerhalb dieses Bereichs wurde kein Einfluß auf den Methioningehalt festgestellt. Bevorzugt ist der Bereich von $10^{-2}$ bis $10^{-4}$ mol/l, falls ein Protein ohne N-terminales Methionin erwünscht ist.

Die zelluläre Aktivität der Methioninabspaltung von Proteinen nach deren Synthese in vivo kann außer durch Wahl geeigneter Fermentationsbedingungen (s. unten) durch Zusätze von bestimmten Metallionen zum Fermentationsmedium nach Wunsch gesteuert werden.

Schwermetalle (vgl. Römpp, Chemielexikon, Stichwort) im Sinne der Erfindung sind vorzugsweise Zink, Kobalt, Nickel, Kupfer und insbesondere Mangan.

Eine Steigerung der Methionin entfernenden Aktivität findet man z.B. ausgeprägt bei Zugabe von Mn-Salzen. Eine Hemmung der Met-Abspaltungsaktivität wird demgegenüber durch die Entfernung dieser Ionen aus dem Medium erreicht, d.h. daß der Entzug von $Mn^{++}$-oder anderen Schwermetallionen eine besonders auffällige Aktivitätshemmung bewirkt und damit ein Protein mit hohem Met-Anteil am N-Terminus entstehen läßt. In diesem Fall liegt der Gehalt an $Mn^{++}$-Ionen vorzugsweise bei $10^{-6}$ oder darunter.

Eine signifikante Abhängigkeit der Met-Abspaltungsaktivität liegt z.B. im Fall der $Mn^{++}$-Ionen im Konzentrationsbereich zwischen $10^{-2}$ und $10^{-6}$, insbesondere bei $10^{-3}$ bis $10^{-4}$ molar je nach sonst gewählten Bedingungen, z .B. Anwesenheit anderer Ionen und sonstiger Medienkomponenten, Temperatur, Konzentration usw., vor.

Einen Einfluß auf den Met-Gehalt des Proteins hat auch der pH-Wert des Mediums: pH-Werte über 7, insbesondere 7 bis 8, fördern die Met-Abspaltungsaktivität und führen zu einem Protein mit geringerem Gehalt an N-ständigem Met. pH-Werte unter 7, insbesondere 5 bis 7, führen zu Produkten mit einem höheren Met-Gehalt. Die Met-Abspaltungs-Aktivität ist auch davon abhängig, welche Aminosäure unmittelbar auf das N-terminale Met folgt. Eine leicht zu spaltende Sequenz, wie z.B. Met-Ala-erfordert eine geringere zelluläre Aktivität des Enzyms, damit Met-freies, am N-Terminus also mit Alanin beginnendes Protein entsteht, als eine schwer zu spaltende Sequenz, wie z.B. Met-Val.

Außerdem wird die Met-abspaltende Aktivität von der Wachstumsgeschwindigkeit der Organismen beeinflußt und vor allem von der momentanen Intensität der Proteinsynthese. Das bedeutet, daß bei hoher heterologer Gen-expression, wenn z.B. mehr als 30% des Gesamtproteins einer Wirtszelle als gewünschtes Fremdprotein synthetisiert wird, eine maximale Aktivität der Met-Abspaltungsreaktion erreicht werden muß, wenn ein Met-freies Fremdprotein isoliert werden soll.

Die besten Bedingungen für die Einstellung des Met-Gehaltes verschiedener Proteine werden daher zweckmäßig jeweils durch Vorversuche ermittelt. Das gleiche gilt für die verwendeten Organismen -sowohl für die verschiedenen verwendeten E. coli-Stämme als auch für Pseudomonaden, Streptomyceten und andere Prokaryonten.

Die für das Verfahren verwendeten Kulturflüssigkeiten basieren vorzugsweise auf der Verwendung der üblichen für die Kultur von Bakterien eingesetzten Komponenten, wie primärem und sekundärem Kaliumphosphat, NaCl, $CaCl_2$, $K_2SO_4$, $MgCl_2$, $NH_3$, Caseinhydrolysaten, Hefeextrakt und Glycerin und Spurenelementen.

Das erfindungsgemäße Verfahren kann verwendet werden, um in Prokaryonten Proteine herzustellen bzw. daraus zu isolieren, die je nach Verwendungszweck einen hohen Anteil an N-ständigem Met enthalten oder aber weitgehend frei von Met am N-Terminus sind. Die Tatsache, daß mit Hilfe des erfindungsgemäßen Verfahrens eine graduelle Steuerung des N-terminalen Met-Gehalts erfolgen kann, erlaubt ihren universellen Einsatz, zum Beispiel

-unabhängig vom Expressionsgrad eines Fremdproteins z.B. in E. coli,

-unabhängig von den gentechnologischen Methoden, Vektoren, Wirtsorganismen, die zur Expression des Proteins eingesetzt werden,

-unabhängig davon, ob mehrere Proteine gleichzeitig oder nur ein Protein hergestellt wird,

-unabhängig vom Verwendungszweck der so hergestellten Proteine (als Pharmazeutika, Diagnostika, Biokatalysatoren usw.), und

-gleichgültig ob native Proteine, Fusionsproteine, Proteinfragmente oder modifizierte Proteine hergestellt werden.

Die im folgenden angeführten Beispiele liefern Anhaltswerte; sie schließen modifizierte Anwendungen des erfindungsgemäßen Verfahrens nicht aus.

Ausführungsbeispiele

1. Ein E. coli K 12-Stamm mit einem Expressionsplasmid, in dem das Gen für den menschlichen Tumornekrose-Faktor (TNF) mit einer Effizienz von 10 bis 60 % des Gesamtproteins exprimiert wird, kann so gesteuert werden, daß der N-terminale Met-Gehalt des TNF sehr hoch ist (mehr als 70 % der TNF-Moleküle N-terminal methioniert) oder aber auf 10 % und weniger abfällt (vgl. Tab.).

Zu diesem Zweck wird ein gemäß Nature 313, 803 (1985) hergestellter, TNF exprimierender E.coli-Stamm in einem Medium (g/l) bestehend aus Glycerin (40), Caseinhydrolysat (20), Hefeextrakt -(5), $KH_2-PO_4$ (5), $NH_4Cl$ (1), $K_2SO_4$ (2), $CaCl_2$, $MgCl_2$ und üblichen Mikronährstoffen 24 h bei 37°C und guter Belüftung und pH-Kontrolle fermentiert.

| Tabelle: | ohne zugesetztes $MnCl_2$ | mit $10^{-3}$ mol/l $MnCl_2$ |
|---|---|---|
| Gehalt an Met-freiem TNF in % | 27 | 89 |

Der N-terminale Met-Gehalt im rekombinanten Protein ist unter entsprechenden Bedingungen z.B. von der Mn$^{++}$-Konzentration im Fermentationsmedium in logarithmisch-proportionalem Verhältnis abhärigig (vgl: Figur).

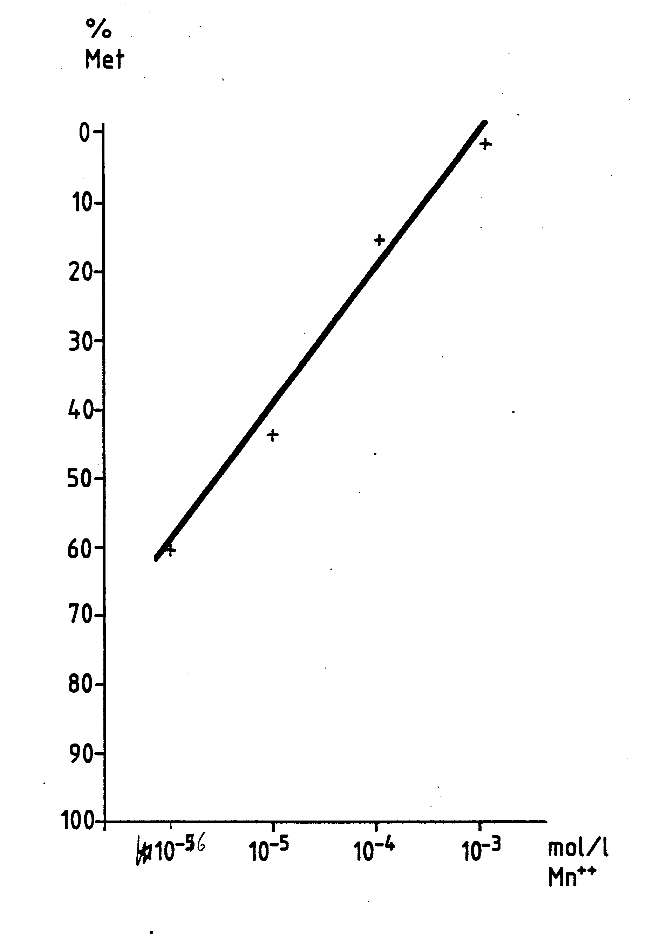

Zur Ermittlung der Abhängigkeit wurde der in 1. beschriebene E.coli-Stamm in LB-Medium mit Zusatz von 20 g/l Glycerin, 10 g/l Caseinhydrolysat, 4 g/l KH$_2$PO$_4$ und üblichen Mikronährstoffen bei pH 7 bis 8 während 18 Stunden bei 37°C gezüchtet. In den einzelnen Versuchen wurde die Konzentration an Mn$^{2+}$ in der aus der Figur erkennbaren Weise variiert und die Analyse des N-ständigen Met mittels HPLC im Phosphatgradienten an entsprechend vorgereinigten Proteinen vorgenommen.

## Ansprüche

1. Verfahren zur Herstellung von Proteinen durch Züchtung gegebenenfalls gentechnologisch veränderter prokaryotischer Mikroorganismen und Gewinnung des gewünschten Proteins aus der Biomasse, wobei das gewünschte Protein am N-terminalen Ende definiert ist im Hinblick auf An-oder Abwesenheit der Aminosäure Methionin, dadurch gekennzeichnet, daß man die Züchtung je nach dem gewünschten Methioningehalt bei einer in Fermentationsmedium gezielt im Bereich von 10$^{-2}$ bis unter 10$^{-6}$ mol/l engestellten Konzentration an Schwermetallionen vornimmt.

2. Verfahren nach Anspruch 1, dadurch gekennzeichnet, daß die Schwermetallionen in Fermentationsmedium in einer Konzentration von 10$^{-2}$ bis 10$^{-4}$ mol/l vorliegen.

3. Verfahren nach Anspruch 1, dadurch gekennzeichnet, daß man Manganionen als Schwermetallionen einsetzt.

4. Verfahren nach Anspruch 2, dadurch gekennzeichnet , daß die Manganionen im Fermentationsmedium in einer Konzentration von 10$^{-2}$ bis 10$^{-4}$ mol/l vorliegen.

5. Verfahren nach Anspruch 1, dadurch gekennzeichnet, daß man zusätzlich den pH-Wert der Lösung auf pH 7 bis 8 für geringen bzw. pH 5 bis 7 für hohen Methioningehalt einstellt.

6. Verfahren zur Steuerung des Gehaltes an N-terminalem Methionin bei der gentechnologischen Herstellung von Proteinen, dadurch gekennzeichnet, daß man den Gehalt an Mn$^{2+}$-Ionen im Medium auf einen Wert zwischen 10$^{-2}$ und praktisch 0 mol/l einstellt.